# FASCICULE DE BREVET EUROPEEN

(11) **EP 0 679 369 B1**
(45) Date de publication et mention de la délivrance du brevet: **21.07.1999**
(21) Numéro de dépôt: 95420107.5
(22) Date de dépôt: 24.04.1995
(51) Int. Cl.: A61B 17/58

(54) **Dispositif de retenue d'une tige de liaison d'un fixateur de rachis sur une vis pédiculaire**
Vorrichtung zur Befestigung eines spinalen Verbindungsstabes an einer Pedikelschraube
Device for attaching a spinal fixation rod to a pedicular screw

(30) Priorité: 25.04.1994 FR 9405189
(43) Date de publication de la demande: 02.11.1995
(73) Titulaire: SOCIETE DE FABRICATION DE MATERIEL ORTHOPEDIQUE SOFAMOR, 93290 Tremblay-en-France (FR)
(72) Inventeur: Fournet-Fayard, Jacques, F-26000 Valence (FR); Garin, Christophe, F-69006 Lyon (FR); Galland, Olivier, F-38240 Meylan (FR); Lucet, Alain, F-21000 Dijon (FR)
(74) Mandataire: Polus, Camille

(56) Documents cités:
- EP-A- 0 452 792
- EP-A- 0 577 219
- FR-A- 2 615 095

## Description

La présente invention a trait à un dispositif permettant la retenue d'une tige de liaison d'un fixateur de rachis sur des vis pédiculaires préalablement ancrées dans les pédicules des vertèbres lombaires d'une colonne vertébrale.

On connait plusieurs types de dispositifs permettant la retenue des tiges de liaison des fixateurs de rachis.

Il existe des dispositifs de retenue qui sont montés sur la tige de liaison avant sa mise en place sur les vis pédiculaires ancrées dans les vertèbres lombaires. Ces dispositifs sont constitués par des bagues présentant un profil interne identique à celui de la tige pour être traversées par cette dernière. Des écrous permettent le serrage de la tige à l' intérieur des bagues. Ces dispositifs de retenue demandent un parfait alignement des vis pédiculaires dans le plan sagittal et frontal. En effet, si l'alignement n'est pas respecté, la réunion et la connexion par la tige est impossible à moins de déformer cette dernière pour récupérer le décalage angulaire. La déformation de la tige ne facilite pas le coulissement et la mise en place des dispositifs de retenue avant la fixation de ladite tige sur les vis pédiculaires.

On connaît aussi par le FR-2 615 095 une instrumentation pour la correction de scolioses lombaires comprenant des vis pédiculaires à double filetage, des tiges associées et des pinces de retenue des tiges par rapport aux vis pédiculaires. D'autres dispositifs de retenue sont montés au fur et à mesure sur les vis pédiculaires ancrées dans les pédicules des vertèbres lombaires. Ces dispositifs exigent de la part du chirurgien une grande dextérité pour monter tous les éléments du fixateur de rachis sur les vis pédiculaires.

Aucun des dispositifs de retenue ci -dessus ne permet aux chirurgiens de choisir la façon de monter le fixateur sur les vis pédiculaires, c' est-à-dire soit la mise en place de la tige de liaison solidaire des dispositifs de retenue, soit un montage au fur et à mesure du déroulement de l'opération.

C'est à ces inconvénients qu'entend plus particulièrement remédier la présente invention.

Les dispositifs de retenue suivant l' invention ont pour but de permettre aux chirurgiens de choisir le montage le mieux adapté du fixateur sur les vis pédiculaires selon les difficultés opératoires rencontrées.

Le dispositif de retenue suivant l'invention comprend deux rondelles identiques comportant chacune des moyens de retenue déformables élastiquement qui permettent de maintenir lesdites rondelles l'une par rapport a l'autre dans différentes positions avant leur assemblage définitif sur la vis pédiculaire correspondante.

Le dispositif de retenue comprend deux rondelles identiques dont chacune comporte un alésage débouchant à profil conique, au moins une encoche cylindrique ménagée perpendiculairement à l'alésage conique pour la mise en place et le guidage d'une tige de liaison, au moins un doigt déformable élastiquement et au moins un trou vertical qui est destiné à recevoir et maintenir le doigt correspondant de l'autre rondelle.

Le dessin annexé, donné à titre d'exemple, permettra de mieux comprendre l'invention, les caractéristiques qu'elle présente et les avantages qu'elle est susceptible de procurer :
Fig. 1 est une vue montrant un pédicule d'une vertèbre lombaire dans lequel est fixée une vis pédiculaire recevant le dispositif de retenue suivant la présente invention.
Fig. 2 est une vue en perspective éclatée illustrant les rondelles constituant le dispositif de retenue suivant la présente invention.
Fig. 3 illustre en coupe le dispositif de liaison entre les rondelles préalablement à leur assemblage.
Fig. 4 et 5 sont des vues montrant la première position d'utilisation du dispositif de retenue.
Fig. 6 et 7 sont des vues semblables à celles des Fig. 4 et 5, mais illustrant la seconde position d'utilisation du dispositif.

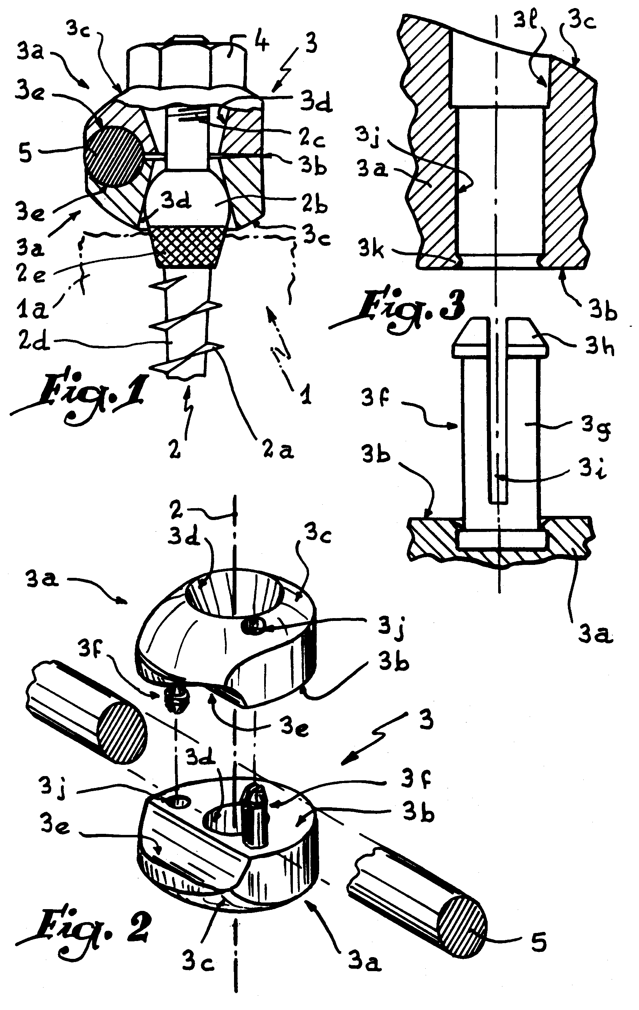

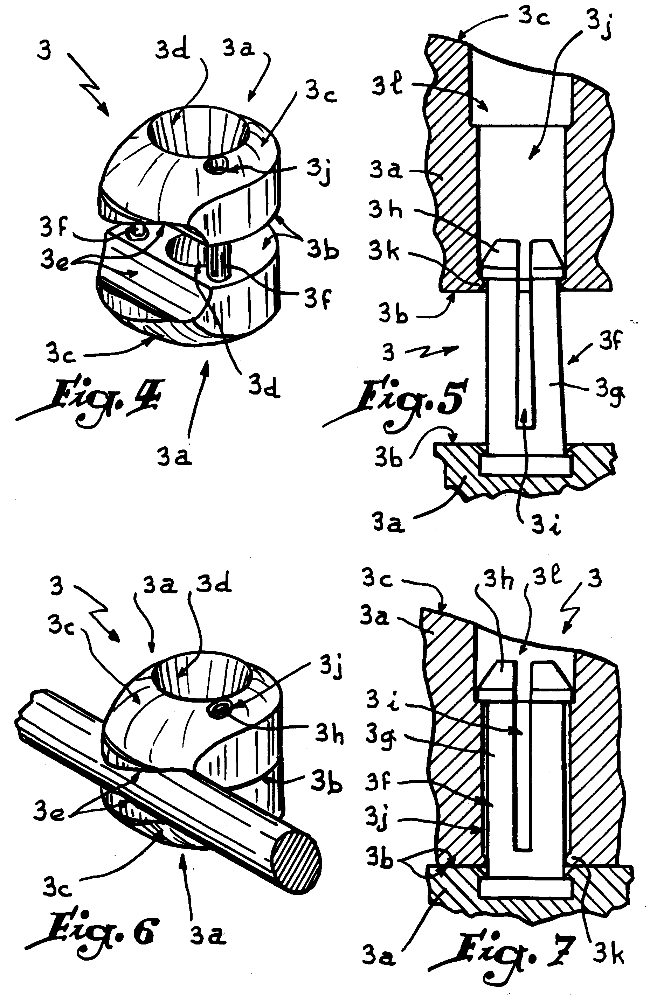

On a représenté en Fig. 1 le pédicule 1a d'une vertèbre lombaire 1 de la partie basse d'une colonne vertébrale dans laquelle est fixée une vis pédiculaire 2. La vis pédiculaire 2 reçoit dans sa partie supérieure un dispositif de retenue 3 qui, par l'intermédiaire d'un écrou 4, permet le serrage d'une tige de liaison 5.

Le fixateur de rachis est composé de plusieurs éléments identiques, comme représenté en Fig. 1, qui sont immobilisés sur les pédicules 1a de chaque vertèbre 1 endommagée de manière à disposer parallèlement aux apophyses épineuses deux tiges de liaison 5.

La vis pédiculaire 2 comprend un filet 2a et une tête sphérique 2b dont la partie supérieure est solidaire d'une tige filetée 2c co-axiale au filet 2a.

Le filet 2a comporte une ame 2d à profil conique, de manière à assurer une meilleure stabilité de la partie supérieure de la vis 2. La base 1a plus large du profil conique de l'âme 2d est prévue du côté de la tête sphérique 2b de la vis pédiculaire 2.

La tête sphérique 2b se prolonge du côté du filet 2a par une partie conique 2e dont l'état de surface est rugueux pour permettre un ancrage biologique progressif et durable pour la reprise de l'os autour de la tête.

La tige filetée 2c est prévue de longueur variable suivant le point d'ancrage de la vis pédiculaire 2. De plus, la tige filetée 2c peut être prévue sécable en fonction de la hauteur utilisée.

En Fig. 2, on a montré le dispositif de retenue 3 qui est constitué de deux rondelles 3a identiques pouvant former réciproquement soit la partie inférieure, soit la partie supérieure du dispositif de retenue. Chaque rondelle 3a présente une surface d'appui plane 3b et un profil extérieur sphérique 3c. Chaque rondelle 3a est percée d'un alésage débouchant 3d qui est décalé latéralement par rapport à l'axe vertical desdites rondelles. L' alésage 3d présente un profil conique dont la base la plus large est tournée du côté du profil sphérique 3c des rondelles 3a.

A proximité de la surface d' appui 3b et orthogonalement à l'axe de l ' alésage conique 3d est prévue une encoche 3e à section partiellement cylindrique qui est orientée comme une corde et débouche sur la face 3b.

Un doigt 3f déformable élastiquement est fixé sur chacune des rondelles 3a du dispositif 3. Le doigt 3f est constitué d'un corps cylindrique 3g et d'une extrémité libre présentant un profil conique 3h dont la base la plus large est prévue d'un diamètre extérieur supérieur à celui du corps 3g. Une fente 3i est usinée au milieu du doigt 3f et sur une partie de sa hauteur pour constituer deux branches parallèles déformables élastiquement (Fig. 3 ).

Le corps cylindrique 3g est fixé dans chaque rondelle 3a soit par sertissage, soit par vissage ou analogue. Le doigt 3f s'étend verticalement et perpendiculairement à la surface d'appui 3b de chaque rondelle 3a.

Le doigt 3f est disposé à proximité de l'alésage conique 3d et de l'encoche 3e.

Un trou cylindrique débouchant 3j est percé dans chacune des rondelles 3a du dispositif 3. Le trou 3j comporte à proximité de la surface d'appui 3b un cordon annulaire 3k. A l'opposé du cordon, c'est-à-dire du côté de la surface sphérique 3c, le trou 3j comporte un épaulement 3l dont le diamètre interne est sensiblement équivalent à celui de la base la plus large du profil conique 3h du doigt 3f.

On remarque que le doigt 3f et le trou 3j de chaque rondelle 3a sont prévus de part et d'autre de l'alésage conique 3d et à proximité de l'encoche 3e.

Le dispositif 3 peut être utilisé de manière classique, c'est-àdire que le chirurgien place une première rondelle 3a sur toutes les vis pédiculaires 2 préalablement ancrées. Ensuite, il dispose la tige de liaison 5 dans l'encoche 3e de la première rondelle 3a avant d'encliqueter la seconde par dessus jusqu'à ce que l'extrémité conique 3h de chaque doigt 3f coopère avec l'épaulement 3l des trous 3j correspondants. Ensuite, le chirurgien visse l'écrou 4 sur la tige 2c de la vis pédiculaire 2 pour définitivement immobiliser la tige de liaison 5 sur les vis pédiculaires 2 et à l'intérieur des dispositifs 3.

On constate, lors du serrage de l'écrou 4, que la partie conique de l'alésage 3d de la rondelle 3a vient en appui contre la tête sphérique 2b en vue de se coincer sur cette dernière, permettant en combinaison avec la tige de liaison 5 de répartir les efforts de serrage afin d'éviter l'entraînement en rotation de la vis pédiculaire 2 à l'intérieur du pédicule 1a de la vertèbre 1.

On a représenté en fig. 4 et 5 la première position du dispositif de retenue 3 qui consiste à introduire les doigts 3f à l'intérieur des trous 3j correspondants de chaque rondelle 3a afin que celles-ci puissent librement se déplacer l'une par rapport à l'autre. Chaque doigt 3f est retenu à l'intérieur du trou 3j correspondant par l'intermédiaire du cordon 3k. Ainsi, le chirurgien prépare préalablement tous les dispositifs de retenue 3, de sorte que les rondelles 3a soient liées l'une à l'autre, mais laissant un jeu suffisant pour le passage de la tige de liaison 5. Après avoir vissé les vis pédiculaires 2 dans les pédicules 1a de chaque vertèbre 1, le chirurgien met en place tous les dispositifs 3 de retenue préalablement préparés sur chacune des vis 2 et soulève uniquement la rondelle supérieure 3a pour permettre la mise en place de la tige de liaison 5. Le chirurgien procède ensuite à l'encliquetage des rondelles 3a de manière à ce que chaque partie conique 3h des doigts 3f coopère avec l'épaulement 3l du trou 3j. Ensuite, le serrage définitif du dispositif 3 et de la tige de liaison 5 est effectué à l'aide de l'écrou 4.

En fig. 6 et 7, on a montré la seconde position d'utilisation du dispositif 3 qui consiste à en positionner un certain nombre sur la tige de liaison 5 avant la mise en place de cette dernière sur les vis pédiculaires 2. En effet, le chirurgien vient disposer tous les dispositifs 3 sur la tige de liaison 5 en encliquetant les rondelles 3a les unes par rapport aux autres au moyen de la tête conique 3h de chaque doigt 3f à l'intérieur des épaulements correspondants 3l.

On note que les dispositifs 3 peuvent coulisser librement le long de la tige 5 de manière que le chirurgien puisse les disposer en face de chaque vis pédiculaire 2 préalablement ancrée dans les pédicules 1a des vertèbres lombaires 1. Cette dernière position du dispositif 3 permet au chirurgien un gain de temps important et lui évite de disposer à l'intérieur de la plaie des éléments de petite taille risquant de lui échapper.

Bien évidemment, le serrage définitif de la tige de liaison 5 est effectué à l'aide de l'écrou 4 qui vient prendre appui sur la portée sphérique 3c de la rondelle 3a supérieure. En effet, l'écrou 4 comprend dans sa partie inférieure une partie concave de même rayon que celui de la partie sphérique 3c des rondelles 3a.

## Revendications

1. Dispositif pour la retenue d'une tige de liaison (5) d'un fixateur de rachis sur des vis pédiculaires (2) d'ancrage dans des pédicules de vertèbres lombaires, caractérisé en ce qu'il comprend deux rondelles identiques (3a) équipées chacune de moyens de retenue déformables élastiquement (3f, 3j) qui permettent de retenir lesdites rondelles l'une par rapport à l'autre et en ce que chaque rondelle (3a) est percée d'un alésage débouchant (3d) pour la fixation du dispositif sur la vis pédiculaire et comporte une encoche (3e) adaptée pour recevoir une tige de liaison.

2. Dispositif suivant la revendication 1, caractérisé en ce que ledit alésage débouchant de chaque rondelle (3d) a un profil conique, et que la dite encoche de chaque rondelle est cylindrique (3e) et est ménagée perpendiculairement à l'alésage conique (3d) pour la mise en place d'une tige de liaison (5), et qu'il est prévu au moins un doigt (3f) déformable élastiquement et au moins un trou vertical (3j) qui est destiné à recevoir et maintenir le doigt (3f) correspondant de l'autre rondelle (3a) avant leur assemblage sur la vis pédiculaire (2) correspondante.

3. Dispositif suivant la revendication 2, caractérisé en ce que chaque rondelle (3a) comporte une surface d'appui (3b) et un profil extérieur sphérique (3c).

4. Dispositif suivant la revendication 2, caractérisé en ce que le doigt (3f) et le trou débouchant (3j) de chaque rondelle (3a) sont prévus perpendiculaires à la surface d'appui (3b).

5. Dispositif suivant la revendication 2 , caractérisé en ce que le doigt (3f) et le trou débouchant (3j) de chaque rondelle (3a) sont prévus de part et d'autre de l'alésage conique (3d) et à proximité de l'encoche cylindrique (3e).

6. Dispositif suivant la revendication 2, caractérisé en ce que le doigt (3f) comporte un corps cylindrique (3g) solidaire de la rondelle (3a) correspondante et dont son extrémité libre présente un profil conique (3h) dont la base la plus large est d'un diamètre extérieur supérieur à celui du corps (3g), tandis que le milieu du doigt (3f) comprend une fente (3i) verticale et disposée sur une grande partie de sa hauteur.

7. Dispositif suivant la revendication 2, caractérisé en ce que le trou débouchant (3j) recevant le doigt (3f) correspondant de chaque rondelle (3a) comprend un cordon (3k) disposé à proximité de la surface plane (3b), tandis que du côté opposé au cordon (3k) c'est-à-dire au voisinage de la surface extérieure sphérique (3c) est prévu un épaulement (3l) dont le diamètre interne est sensiblement équivalent à celui de la base la plus large du profil conique (3h) du doigt (3f).

8. Dispositif suivant la revendication 2, caractérisé en ce que le diamètre du trou (3j) est inférieur à celui de la base la plus large du cône (3h) de manière à créer un effort élastique sur le doigt (3f).

9. Dispositif suivant la revendication 2, caractérisé en ce que la base la plus large de l'alésage conique (3d) est tournée du côté du profil extérieur sphérique (3c) de chaque rondelle (3a).

10. Dispositif suivant la revendication 2, caractérisé en ce que le diamètre des encoches (3e) est équivalent à celui de la tige de liaison (5) de manière à serrer cette dernière sur tout son pourtour.

11. Dispositif suivant la revendication 1, caractérisé en ce que chaque rondelle (3a) présente un alésage conique (3d) destiné à prendre appui contre une partie sphérique (2b) de la vis pédiculaire (2) en vue de son coincement, permettant en combinaison avec la tige de liaison (5) de répartir les efforts de serrage engendrés par l'écrou (4) afin d'éviter l'entraînement en rotation de la vis pédiculaire (2) à l'intérieur des pédicules (1a).

## Claims

1. Device for attaching a spiral fixator connecting rod (5) to pedicle screws (2) for anchorage in lumbar vertebra pedicles, characterized in that it comprises two identical washers (3a), each equipped with elastically deformable attachment means (3f, 3j) which allow the said washers to be attached to one another, and in that each washer (3a) is pierced by an open bore (3d) for fixing the device on the pedicle screw and comprises a cut-out (3e) designed to accommodate a connecting rod.

2. Device according to Claim 1, characterized in that the said open bore (3d) of each washer has a conical profile, and that the said cut-out of each washer is cylindrical (3e) and is formed at right angles to the conical bore (3d) for the insertion of a connecting rod (5), and that there is at least one elastically deformable finger (3f) and at least one vertical hole (3j) which is intended to accommodate and hold the corresponding finger (3f) of the other washer (3a) before they are assembled onto the corresponding pedicle screw (2).

3. Device according to Claim 2, characterized in that each washer (3a) comprises a bearing surface (3b) and a spherical exterior profile (3c).

4. Device according to Claim 2, characterized in that the finger (3f) and the open hole (3j) of each washer (3a) are made at right angles to the bearing surface (3b).

5. Device according to Claim 2, characterized in that the finger (3f) and the open hole (3j) of each washer (3a) are provided one on each side of the conical bore (3d) and near to the cylindrical cut-out (3e).

6. Device according to Claim 2, characterized in that the finger (3f) comprises a cylindrical body (3g) integral with the corresponding washer (3a) and the free end of which has a conical profile (3h), the widest base of which has an outside diameter that exceeds that of the body (3g), while the middle of the finger (3f) has a vertical slot (3i) over a significant proportion of its height.

7. Device according to Claim 2, characterized in that the open hole (3j) accommodating the corresponding finger (3f) of each washer (3a) comprises a bulge (3k) arranged near the planar surface (3b), while at the opposite end to the bulge (3k), that is to say near to the spherical exterior surface (3c), there is a shoulder (3l), the inside diameter of which is roughly equivalent to the diameter of the widest base of the conical profile (3h) of the finger (3f).

8. Device according to Claim 2, characterized in that the diameter of the hole (3j) is smaller than that of the widest base of the cone (3h), so as to create an elastic force on the finger (3f).

9. Device according to Claim 2, characterized in that the widest base of the conical bore (3d) faces towards the spherical exterior profile (3c) of each washer (3a).

10. Device according to Claim 2, characterized in that the diameter of the cut-outs (3e) is equivalent to that of the connecting rod (5) so as to clamp this rod over its entire periphery.

11. Device according to Claim 1, characterized in that each washer (3a) has a conical bore (3d) intended to bear against a spherical part (2b) of the pedicle screw (2) with a view to wedging it, making it possible, in combination with the connecting rod (5), to spread the clamping forces generated by the nut (4) so as to prevent the pedicle screw (2) from being made to turn inside the pedicles (1a).

## Patentansprüche

1. Vorrichtung zur Halterung eines Verbindungsstabs (5) eines spinalen Fixiermittels auf Pedikelschrauben (2) zur Verankerung in Lendenwirbelpedikeln, dadurch gekennzeichnet, daß sie zwei identische Unterlegscheiben (3a) aufweist, die jeweils mit einer elastisch verformbaren Halterungseinrichtung (3f, 3j) versehen sind, die es erlauben, die Unterlegscheiben in Bezug aufeinander zu halten, und daß in jede Unterlegscheibe (3a) eine Durchlaßbohrung (3d) zur Fixierung der Vorrichtung auf der Pedikelschraube eingebracht ist und eine Kerbe (3e) aufweist, die dazu ausgelegt ist, einen Verbindungsstab aufzunehmen.

2. Vorrichtung nach Anspruch 1, dadurch gekennzeichnet, daß die Durchlaßbohrung (3d) jeder Unterlegscheibe ein konisches Profil aufweist und daß die Kerbe (3e) jeder Unterlegscheibe zylindrisch und lotrecht zu der konischen Bohrung (3d) zum Anbringen eines Verbindungsstabs (5) vorgesehen ist, und daß sie mit zumindest einem elastisch verformbaren Zapfen (3f) und zumindest einem vertikalen Loch (3j) versehen ist, das dazu bestimmt ist, den entsprechenden Zapfen (3f) der anderen Unterlegscheibe (3a) vor ihrer Anbringung auf der entsprechenden Pedikelschraube (2) aufzunehmen und zu halten.

3. Vorrichtung nach Anspruch 2, dadurch gekennzeichnet, daß jede Unterlegscheibe (3a) eine Anlagefläche (3b) und ein kugelförmiges Außenprofil (3c) aufweist.

4. Vorrichtung nach Anspruch 2, dadurch gekennzeichnet, daß der Zapfen (3f) und das Durchgangsloch (3j) jeder Unterlegscheibe (3a) lotrecht zur Anlagefläche (3b) vorgesehen sind.

5. Vorrichtung nach Anspruch 2, dadurch gekennzeichnet, daß der Zapfen (3f) und das Durchgangsloch (3j) jeder Unterlegscheibe (3a) beiderseits der konischen Bohrung (3d) sowie neben der zylindrischen Kerbe (3e) vorgesehen sind.

6. Vorrichtung nach Anspruch 2, dadurch gekennzeichnet, daß der Zapfen (3f) einen zylindrischen Körper (3g) aufweist, der mit der entsprechenden Unterlegscheibe (3a) durchgehend bzw. einstückig vorgesehen ist und dessen freies Ende ein konisches Profil (3h) aufweist, dessen größte Basis einen Außendurchmesser größer als derjenige des Körpers (3g) aufweist, während die Mitte des Zapfens (3f) einen Schlitz (3i) aufweist, der vertikal verläuft und über einen großen Teil seiner Höhe angeordnet ist.

7. Vorrichtung nach Anspruch 2, dadurch gekennzeichnet, daß das den entsprechenden Zapfen (3f) jeder Unterlegscheibe (3a) aufnehmende Durchgangsloch (3j) einen Wulst (3k) aufweist, der in der Nähe der ebenen Oberfläche (3b) angeordnet ist, während die Seite in Gegenüberlage zu dem Wulst (3k), d.h. benachbart zu der kugelförmigen Außenfläche (3c), mit einer Schulter (31) versehen ist, deren Innendurchmesser im wesentlichen gleich demjenigen der größten Basis des konischen Profils (3h) des Zapfens (3f) ist.

8. Vorrichtung nach Anspruch 2, dadurch gekennzeichnet, daß der Durchmesser des Lochs (3j) kleiner als derjenige der größten Basis des Konus (3h) derart ist, daß auf dem Zapfen (3f) eine elastische Kraft erzeugt ist.

9. Vorrichtung nach Anspruch 2, dadurch gekennzeichnet, daß die größte Basis der konischen Bohrung (3d) zur Seite des kugelförmigen Außenprofils (3c) jeder Unterlegscheibe (3a) drehversetzt ist.

10. Vorrichtung nach Anspruch 2, dadurch gekennzeichnet, daß der Durchmesser der Kerben (3e) gleich demjenigen des Verbindungsstabs (5) derart ist, daß letztgenannte auf ihrem gesamten Außenumfang eingespannt ist.

11. Vorrichtung nach Anspruch 1, dadurch gekennzeichnet, daß jede Unterlegscheibe (3a) eine konische Bohrung (3d) aufweist, die dazu bestimmt ist, an einem kugelförmigen Teil (2b) der Pedikelschraube (2) zu ihrer Klemmung anliegt, die es in Verbindung mit dem Verbindungsstab (5) erlaubt, die durch die Schraube (4) erzeugte Einspannung abzuführen, um die Drehmitnahme der Pedikelschraube (2) in das Innere der Pedikel (1a) zu vermeiden.
